# EUROPÄISCHE PATENTSCHRIFT

(11) **EP 2 167 641 B1**
(45) Veröffentlichungstag und Bekanntmachung des Hinweises auf die Patenterteilung: **06.05.2015**
(21) Anmeldenummer: 08759076.6
(22) Anmeldetag: 06.06.2008
(51) Int. Cl.: C12N 5/00

(54) **VERFAHREN UND VORRICHTUNG ZUR BILDUNG EINER DREIDIMENSIONALEN ANORDNUNG BIOLOGISCHER ZELLEN**
METHOD AND DEVICE FOR FORMING A THREE-DIMENSIONAL ARRANGEMENT OF BIOLOGICAL CELLS
PROCÉDÉ ET DISPOSITIF PERMETTANT DE FORMER UN ENSEMBLE TRIDIMENSIONNEL DE CELLULES BIOLOGIQUES

(30) Priorität: 20.06.2007 DE 102007028422
(43) Veröffentlichungstag der Anmeldung: 31.03.2010
(73) Patentinhaber: Fraunhofer-Gesellschaft zur Förderung der angewandten Forschung e.V., 80686 München (DE)
(72) Erfinder: KRUSE, Charli, 23923 Herrnburg (DE); CIBA, Philipp, 23552 Lübeck (DE); FUHR, Günter, 13187 Berlin (DE)
(74) Vertreter: Hertz, Oliver
(86) Internationale Anmeldenummer: PCT/EP2008/004538
(87) Internationale Veröffentlichungsnummer: WO 2008/155028

(56) Entgegenhaltungen:
- WO-A-02/18937
- BALABAN NATHALIE Q ET AL: "Force and focal adhesion assembly: A close relationship studied using elastic micropatterned substrates" NATURE CELL BIOLOGY, Bd. 3, Nr. 5, Mai 2001 (2001-05), Seiten 466-472, XP002502427 ISSN: 1465-7392
- BISCHOFS I B ET AL: "Cell organization in soft media due to active mechanosensing." PROCEEDINGS OF THE NATIONAL ACADEMY OF SCIENCES OF THE UNITED STATES OF AMERICA, Bd. 100, Nr. 16, 5. August 2003 (2003-08-05), Seiten 9274-9279, XP002502428 ISSN: 0027-8424
- HARRIS A K: "Tissue culture cells on deformable substrata: biomechanical implications" JOURNAL OF BIOMECHANICAL ENGINEERING, NEW YORK, NY, Bd. 106, Nr. 1, 1. Februar 1984 (1984-02-01), Seiten 19-24, XP009107251 ISSN: 0148-0731
- LO CHUN-MIN ET AL: "Cell movement is guided by the rigidity of the substrate" BIOPHYSICAL JOURNAL, Bd. 79, Nr. 1, Juli 2000 (2000-07), Seiten 144-152, XP002502429 ISSN: 0006-3495
- CURTIS ADAM ET AL: "Measuring cell forces by a photoelastic method" BIOPHYSICAL JOURNAL, Bd. 92, Nr. 6, März 2007 (2007-03), Seiten 2255-2261, XP002502430 ISSN: 0006-3495
- BRANDL ET AL: "Rational design of hydrogels for tissue engineering: Impact of physical factors on cell behavior" BIOMATERIALS, ELSEVIER SCIENCE PUBLISHERS BV., BARKING, GB, Bd. 28, Nr. 2, 1. Januar 2007 (2007-01-01), Seiten 134-146, XP005706249 ISSN: 0142-9612
- NICOLAS ALICE ET AL: "Dynamics of cellular focal adhesions on deformable substrates: consequences for cell force microscopy" BIOPHYSICAL JOURNAL, NEW YORK, US, US, Bd. 95, Nr. 2, 1. Juli 2008 (2008-07-01), Seiten 527-539, XP009106953 ISSN: 0006-3495
- YANG Z ET AL: "Determining substrate displacement and cell traction fields-a new approach", JOURNAL OF THEORETICAL BIOLOGY, ACADEMIC PRESS, LONDON, GB, vol. 242, no. 3, 7 October 2006 (2006-10-07), pages 607-616, XP024953700, ISSN: 0022-5193, DOI: 10.1016/J.JTBI.2006.05.005 [retrieved on 2006-10-07]

## Beschreibung

Die Erfindung betrifft ein Verfahren zur Bildung einer dreidimensionalen Zellanordnung mit einer Vielzahl biologischer Zellen, insbesondere ein Verfahren zur Einstellung oder Veränderung einer geometrischen Raumform einer Zellanordnung und Verfahren zur geometrischen Strukturierung von Zellmaterial, wie z. B. Verfahren zum sog. Tissue Engineering. Die Erfindung betrifft auch eine Vorrichtung zur Durchführung dieser Verfahren, insbesondere ein Substrat für eine Zellanordnung, wie z. B. eine Zellkultur oder Gewebe, mit dem die geometrische Raumform einer Zellanordnung einstellbar oder veränderbar ist. Die Erfindung betrifft auch Anwendungen des Verfahrens und der Vorrichtung.

In der Zellbiologie werden biologische Zellen typischerweise in einem adhärenten Zustand auf einem ebenen Substrat einer Kultivierung (Wachstum und/oder Differenzierung) unterzogen. Die Kultivierung auf dem Substrat hat den Vorteil, dass eine ausreichende Versorgung der Zellen mit Nährstoffen und eine Beobachtung der Zellen, z. B. mit einem Mikroskop ermöglicht werden. Nachteilig ist jedoch, dass der adhärente Zustand auf einem Substrat nicht den natürlichen Lebensbedingungen der Zellen entspricht. Im lebenden Organismus, insbesondere im lebenden tierischen oder menschlichen Organismus bilden die Zellen dreidimensionale Zellanordnungen, wie z. B. Gewebe oder Organe. In einer dreidimensionalen Zellanordnung sind durch die Bildung von Zell-Zell-Kontakten im Raum andere Lebensbedingungen der Zellen gegeben, als auf einem ebenen Substrat. Es besteht daher das Interesse, insbesondere für Zwecke der in *vitro*-Untersuchung biologischer Zellen oder der medizinischen Zelltherapie oder Gewebebearbeitung, insbesondere dem Tissue Engineering, dreidimensionale Zellanordnungen synthetisch nachzubilden.

In WO 2004/046337 wird vorgeschlagen, eine dreidimensionale Zellanordnung durch eine Struktur aus Zell- und Biopolymerschichten nachzubilden. In dieser Struktur sind die Zellen im Biopolymer räumlich verteilt. Diese Technik hat den Nachteil, dass nur beschränkt natürliche Lebensbedingungen der Zellen geschaffen werden können. Die Biopolymere bilden nicht die typische Umgebung der Zellen im lebenden Organismus. Weitere Nachteile bestehen in der beschränkten Variabilität bei der Formgebung für die Zellanordnung und der beschränkten Versorgung der im Biopolymer eingebetteten Zellen mit Nährstoffen.

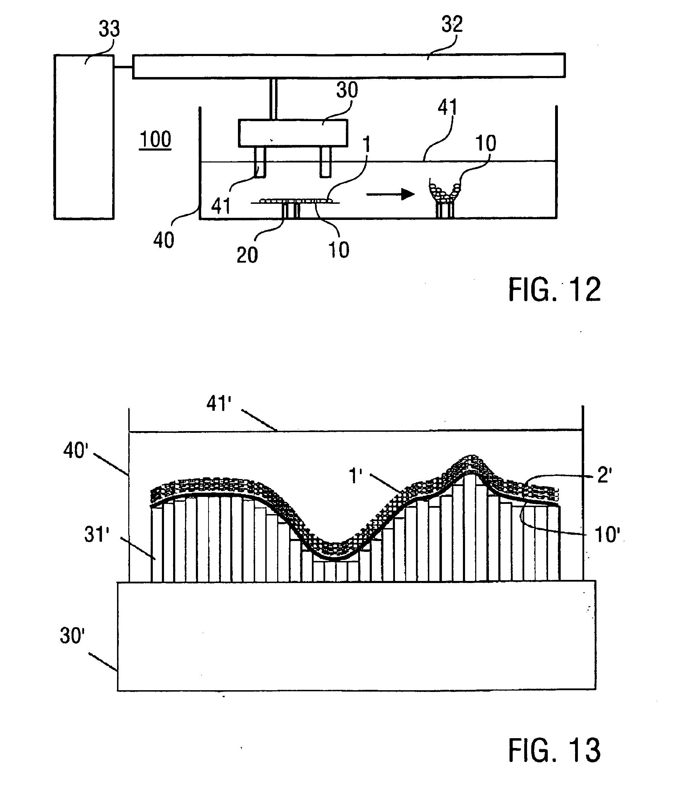

Aus WO 2004/074425 ist ein Verfahren zur geometrischen Formung von Zellmaterial unter Verwendung eines verstellbaren Manipulationswerkzeugs bekannt. Eine Variante dieses Verfahrens ist in Figur 13 gezeigt. Das Zellmaterial 1' mit einer Vielzahl biologischer Zellen 2' ist auf einem schichtförmigen, biegsamen Substrat 10' angeordnet. Die Form des Substrats 10' ist mit einer Antriebseinrichtung 30' und einer Vielzahl von Formelementen 31' veränderbar. Das Substrat 10' mit der Zellanordnung 1' ist in einem Kultivierungsgefäß 40' angeordnet, das eine Kultivierungsflüssigkeit 41' enthält. Durch eine Verschiebung der Formelemente 31' kann die Form des Substrats 10' eingestellt werden, so dass das Zellmaterial mit einer gekrümmten Oberfläche geformt werden kann.

Die praktische Anwendung der in WO 2004/074425 beschriebenen Technik kann wegen der folgenden Nachteile beschränkt sein. Erstens ist die Gestaltungsfreiheit durch die Bewegung der Formelemente 31' beschränkt. Mit der Antriebseinrichtung 30' können bspw. keine Nischen oder Hohlräume geschaffen werden, die jedoch für die Nachbildung natürlicher Kultivierungsbedingungen von hohem Interesse sind. Des Weiteren können die Zellen nur auf der oberen Substratseite wachsen, da die untere Substratseite notwendigerweise von den Formelementen 31' berührt wird. Daraus ergeben sich Beschränkungen für die Versorgung der Zellen mit Nährstoffen. Des Weiteren kann das Substrat eine Störung der natürlichen Kultivierungsbedingungen der Zellen im Zellmaterial darstellen.

Es ist aus der Zellbiologie bekannt, dass Zellen auf Substrate Attraktionskräfte ausüben. In US 2004/0033482 A1 wird vorgeschlagen, die Attraktionskräfte zu messen, indem Zellen auf einem vorgespannten, gekrümmten Substrat kultiviert werden. Durch die Attraktionskräfte wird das Substrat entgegen der Krümmung zurückgebogen, wobei die Kompensation der Krümmung als Maß für die Attraktionskräfte ausgewertet wird.

Die Wechselwirkung adhärenter Zellen mit Substratoberflächen, die insbesondere zu einer Deformation biegsamer Substrate führen kann, wird von N. Q. Balaban et al. in "Nature Cell Biology", Band 3, 2001, S. 466-472, in WO 02/18937 A1, von I. B. Bischofs et al. in "PNAS", Band 100, 2003, S. 9274-9279, von A. K. Harris in "Journal of Biomechanical Engineering", Band 106, 1984, S. 19-24, von C.-M. Lo et al. in "Biophysical Journal", Band 79, 2000, S. 144-152, von A. Curtis et al. in "Biophysical Journal", Band 92, 2007, S. 2255-2261, von F. Brandl et al. in "Biomaterials", Band 28, 2007, S. 134-146, und von A. Nicolas et al. in "Biophysical Journal", Band 95, 2008, S. 527-539 beschrieben.

Die Aufgabe der Erfindung ist es, ein verbessertes Verfahren zur Bildung einer dreidimensionalen Zellanordnung aus biologischen Zellen bereitzustellen, mit dem Nachteile der herkömmlichen Techniken vermieden werden und das insbesondere eine verbesserte Nachbildung natürlicher Lebensbedingungen von Zellen ermöglicht. Des Weiteren ist es die Aufgabe der Erfindung, eine verbesserte Vorrichtung zur Bildung einer dreidimensionalen Zellanordnung zu schaffen, mit der Beschränkungen der herkömmlichen Techniken überwunden werden und die insbesondere einen erweiterten Anwendungsbereich hat.

Diese Aufgaben werden mit einem Verfahren und einer Vorrichtung mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt basiert die Erfindung insbesondere auf der technischen Lehre, eine dreidimensionale Zellanordnung zu formen, indem biologische Zellen auf einem biegsamen Substrat angeordnet werden und auf dieses eine Kraft (Attraktionskraft) ausüben. Die Erfinder haben festgestellt, dass adhärente Zellen unmittelbar auf die Substratoberfläche eine mechanische Spannung ausüben, unter deren Wirkung das biegsame Substrat verformt wird. Diese endogene Attraktionskraft der Zellen wird durch Druck- und/oder Zugkräfte gebildet, die durch das innere Zellskelett und zwischenmolekulare Wechselwirkungen (Bindungen) zwischen Oberflächenmolekülen der Zellen und der Substratoberfläche verursacht werden.

Die Attraktionskraft hat sich als ausreichend stark erwiesen, um biegsame Substrate zu verformen. Obwohl bereits bekannt ist, dass sich Zellen aufgrund der zwischenmolekularen Wechselwirkung auf Substratoberflächen bewegen können, stellt die erfindungsgemäße Substratdeformation ein überraschendes Ergebnis dar, da mit den Zellkräften nicht nur Zellen (d. h. mikroskopisch kleine Partikel), sondern das Substrat mit makroskopischen Dimensionen deformiert wird. So beträgt die von einer Zelle auf ein Substrat ausgeübte Kraft pro Bindung 1 pN bis 5 pN. Es können einige Tausend, z. B. 2000 bis eine Millionen oder mehrere Millionen Bindungen gebildet sein, so dass die Attraktionskraft pro Zelle 1 nN bis 1 µN beträgt. Mit tausend oder mehr Zellen auf dem Substrat beträgt die Attraktionskraft z. B. bis zu 1 mN.

Gemäß einem zweiten Gesichtspunkt der Erfindung wird die genannte Aufgabe insbesondere durch die technische Lehre gelöst, ein Substrat aus einem biegsamen Material bereitzustellen, dessen Substratoberfläche zur Aufnahme biologischer Zellen eingerichtet ist, wobei die Substratoberfläche zur Bildung zwischenmolekularer Wechselwirkungen (molekularer Bindungen) mit den Zellen und zur Aufnahme der von den Zellen auf das Substrat ausgeübten Attraktionskraft derart eingerichtet ist, dass das Substrat unter der Wirkung der Attraktionskraft deformierbar ist. Durch die Wirkung der Attraktionskraft auf das Substrat ist dieses deformierbar. Mit der Deformation des Substrats wird die Zellanordnung im Raum geformt.

Die Nachgiebigkeit bezeichnet allgemein die Eigenschaft eines Körpers, einer Kraft (Zug oder Druck) durch eine Formänderung nachzugeben. Das erfindungsgemäße Substrat weist eine derart geringe Nachgiebigkeit auf, dass das Substrat unter der Wirkung der Attraktionskraft der Zellen insbesondere plastisch oder elastisch deformierbar ist. Das erfindungsgemäße Substrat ist zumindest in Teilbereichen aus einem Material gebildet, in dem eine der Deformation entgegengesetzte Kraft (insbesondere innere Reibungskraft oder elastische Rückstellkraft) geringer als ist die Attraktionskraft. Im Fall einer elastischen Deformierbarkeit hat das Substrat eine derart geringe Biegesteifigkeit, dass es unter der Wirkung der Attraktionskraft deformierbar ist. Die Deformation umfasst insbesondere eine zunehmende Krümmung des Substrats.

Die Verformung des Substrates wird insbesondere dadurch verursacht, dass die Attraktionskraft an verschiedenen Angriffspunkten mit verschiedenen Orientierungen wirkt (Spannung). In Abhängigkeit von den Richtungen der Attraktionskraft wird das Substrat z. B. verzerrt. Die Substratoberfläche des erfindungsgemäßen Substrats weist eine Vielzahl von Kraftangriffspunkten auf, die zur Einwirkung der Attraktionskraft eingerichtet sind. Die Kraftangriffspunkte sind vorzugsweise als lokal begrenzte Teile der Substratoberfläche gebildet.

Die Kraftangriffspunkte (Kraftangriffsbereiche) sind lokale Oberflächenbereiche, die für eine verstärkte zwischenmolekulare Wechselwirkung zwischen den Zellen und der Substratoberfläche und damit zu einer lokalen Ausübung der Attraktionskraft eingerichtet sind. Kraftangriffspunkte zeichnen sich durch eine im Vergleich zur übrigen Substratoberfläche erhöhte Zellhaftung aus. Hierzu ist beispielsweise eine Textur der Substratoberfläche oder eine chemische Modifizierung der Substratoberfläche vorgesehen. Die Kraftangriffspunkte umfassen insbesondere Haftstrukturen und/oder Haftschichten, die mit einer vorbestimmten geometrischen Verteilung in oder auf der Substratoberfläche angeordnet sind. Haftstrukturen werden z. B. durch Kanten, Stufen und/oder Löcher in der Substratoberfläche, wie z. B. Gitterelemente gebildet. Haftschichten umfassen z. B. Fibronektin, Kollagen oder andere Biomaterialien oder eine mit Makromolekülen funktionalisierte Oberflächenbedeckungen.

Mit dem Begriff "Zellanordnung" wird hier eine Zusammensetzung aus einer Vielzahl biologischer Zellen gleicher oder verschiedener Zelltypen bezeichnet, die auf der Oberfläche des Substrats verteilt angeordnet ist. Die Zellanordnung umfasst eine geschlossene Zellschicht auf der Substratoberfläche oder alternativ eine Anordnung von Zellgruppen mit gegenseitigen Abständen. Gemäß einer bevorzugten Ausführungsform der Erfindung bilden die Zellen die geschlossene Zellschicht, wobei die Attraktionskraft von den Zellen unmittelbar auf die Substratoberfläche übertragen wird. In diesem Fall ergibt sich vorteilhafterweise eine besonders effektive Deformation des Substrats. Eine Zellschicht kann z. B. eine einzelne Monolage oder mehrere Zell-Lagen (mindestens zwei Monolagen) umfassen. Bevorzugte Anwendungen der erfindungsgemäß geformten dreidimensionalen Zellanordnung sind in der medizinischen Therapie und bei technischen Applikationen biologischer Zellen gegeben.

Die Erfindung ergibt die folgenden Vorteile. Durch die Formung der Zellanordnung mit dem Substrat wird die Herstellung definierter dreidimensionaler multizellulärer Strukturen ermöglicht. Das Substrat wird deformiert, indem die Zellen auf der Substratoberfläche mechanische Arbeit verrichten. Die Geschwindigkeit der Substratdeformation wird durch die Geschwindigkeit der Umverteilung von molekularen Bindungen zwischen den Zellen und der Substratoberfläche bestimmt. Diese Geschwindigkeit entspricht im Wesentlichen der Rate der natürlichen Bewegung adhärenter Zellen. Die Geschwindigkeit der Substratdeformation wird somit durch physiologische Zelleigenschaften bestimmt, so dass ein störender Einfluss der Substratdeformation auf die Zellanordnung vermieden werden kann.

Vorteilhaft ist ferner, dass die Substratdeformation allgemein ohne eine flächige Berührung des Substrats mit einem Deformationswerkzeug, wie zum Beispiel den herkömmlichen Formelementen, erfolgen kann. Die Ausübung der Attraktionskraft erfolgt ohne eine Berührung des Substrats durch ein mechanisches Werkzeug. Somit kann das Substrat mit einer komplexeren Volumentopographie als bei den herkömmlichen Techniken gebildet werden. Außerdem können Zellen gleicher oder verschiedener Zelltypen auf allen Seiten des Substrats angeordnet sein.

Ein weiterer Vorteil ergibt sich aus der Variabilität der erfindungsgemäß einstellbaren Substratformen. Beispielsweise kann gemäß einer ersten Variante eine Krümmung des Substrats derart vorgesehen sein, dass eine schichtförmige Zellanordnung mit einer Krümmung im Raum gebildet wird. Die schichtförmige Zellanordnung (Zellschicht) kann eine Monoschicht oder eine Mehrfachschicht der biologischen Zellen umfassen, wobei die geometrische Erstreckung der Zellschicht durch eine im Raum gekrümmte Fläche repräsentiert wird. Vorteilhafterweise können damit dreidimensionale Zellstrukturen nachgebildet werden, wie sie im lebenden Organismus auftreten, wobei eine freie Nährstoffversorgung auf der gesamten gekrümmten Oberfläche der Zellschicht gewährleistet ist. Gemäß einer zweiten Variante kann eine Faltung des Substrats vorgesehen sein, bei der das Substrat so stark gekrümmt wird, dass sich mindestens zwei Teilbereiche der Substratoberfläche gegenüberstehen. Der senkrechte Abstand der Teilbereiche der Substratoberfläche wird so gering, dass die Zellen auf den Teilbereichen sich gegenseitig berühren oder den Abstand durch Zellwachstum schließen können. Mit der Substratfaltung bildet die Zellanordnung einen dreidimensionalen Zellhaufen. Im Unterschied zu der herkömmlichen Bildung von Zell- und Biopolymerschichten kann der dreidimensionale Zellhaufen eine lockere Zusammensetzung darstellen. Der Zellhaufen ist für gasförmige und flüssige Medien, insbesondere für die Nährstoffzufuhr durchlässig. Gemäß einer weiteren Variante können die im Raum gekrümmte Zellschicht und der dreidimensionale Zellhaufen kombiniert werden, indem ein Teil des Substrats gekrümmt und ein anderer Teil des Substrats gefaltet wird.

Zur Bildung der Kraftangriffspunkte weist das erfindungsgemäße Substrat vorzugsweise zumindest in einem Teilbereich eine Gitterstruktur (Gerüststruktur) mit einer Vielzahl von Gitterelementen auf. Die Kraftangriffspunkte des erfindungsgemäßen Substrats mit der Gitterstruktur (im folgenden: Gitter-Substrat) sind an den Gitterelementen gebildet. Die Erfinder haben festgestellt, dass auch die Gitterstruktur eine Substratoberfläche bietet, auf der eine geschlossene Zellanordnung gebildet werden kann, welche Löcher der Gitterstruktur überspannt und die Attraktionskraft der Zellen besonders wirksam ausübt. Die Löcher sind zwischen den Gitterelementen gebildet. Die Löcher können eine charakteristische Dimension (zum Beispiel Seitenlänge, Durchmesser) aufweisen, die größer als die Größe einer einzelnen Zelle ist. Das Gitter-Substrat kann somit eine Gitterstruktur aufweisen, die ein vollständiges Umwachsen des Substrats mit den Zellen ermöglicht.

Die Gitterstruktur ist nicht zwingend vorgesehen. Wenn alternativ eine geschlossene Substratoberfläche mit Unterbrechungen, zum Beispiel Gräben oder Vertiefungen, vorgesehen ist, ergeben sich ebenfalls Vorteile für die Bildung der Kraftangriffspunkte.

Alternativ oder zusätzlich weist das erfindungsgemäße Substrat zumindest in einem Teilbereich eine geschlossene Substratoberfläche auf, auf der die Kraftangriffspunkte gebildet sind. Mit der geschlossenen Substratoberfläche, die ohne Ausübung der Attraktionskraft vorzugsweise eben ist, ergeben sich Vorteile für die Bildung einer vollständig abdeckenden Zellanordnung und damit einer erhöhten Wirksamkeit der Attraktionskraft der Zellen.

Geschlossene Substratoberflächen haben vorteilhafte Anwendungen als Abdeckungen für Implantations- oder Wundheilungszwecke. Mit dem erfindungsgemäßen Substrat kann z. B. eine Wundabdeckfolie bereitgestellt werden, die eine Krümmung entsprechend der Oberfläche der zu bedeckenden Wunde aufweist und eine Zellanordnung (z. B. aus Keratinozyten, Hautstammzellen, Endothelzellen, Epithelzellen, aus Stammzellen differenzierte Hautzellen und/oder andere gewebetypische Zellen) trägt, mit der die Wundheilung beschleunigt wird.

Gemäß einer bevorzugten Ausführungsform der Erfindung ist eine gerichtete Deformation des Substrats vorgesehen, bei der die Substratoberfläche gemäß mindestens einer vorbestimmten Hauptdeformationsrichtung gekrümmt wird. Das Substrat hat mindestens eine Vorzugsrichtung der Deformation, d. h. das Substrat hat in mindestens einer Richtung eine geringere Biegesteifigkeit als in anderen Richtungen. Vorteilhafterweise kann die Form der erfindungsgemäß geformten Zellanordnung durch eine Gestaltung (Architektur) des Substrates gezielt bestimmt werden. Die mindestens eine Hauptdeformationsrichtung wird vorzugsweise durch die geometrische Verteilung von vorbestimmten Deformationsbereichen des Substrats und/oder der Kraftangriffspunkte definiert. Vorteilhafterweise kann damit die Art der Zellanordnung (gekrümmte Zellschicht oder dreidimensionaler Zellhaufen) und deren geometrische Form in Abhängigkeit von der konkreten Anwendung der Erfindung eingestellt werden.

Zur Bildung der mindestens einen Hauptdeformationsrichtung weist das erfindungsgemäße Substrat vorzugsweise eine anisotrope und/oder örtliche veränderliche Nachgiebigkeit (insbesondere Biegesteifigkeit) auf. Das Substrat besitzt vorbestimmte Deformationsbereiche erhöhter oder verminderter Biegesteifigkeit. Die Deformationsbereiche umfassen Biege- oder Versteifungsbereiche, in denen das Substrat durch eine geeignete Wahl des Substratmaterials oder der Substratstruktur jeweils entsprechend eine verminderte oder eine erhöhte Biegesteifigkeit aufweist. Alternativ oder zusätzlich zur Bereitstellung der Deformationsbereiche kann die Hauptdeformationsrichtung durch eine unregelmäßige geometrische Verteilung der Kraftangriffspunkte auf der Substratoberfläche bestimmt werden.

Das erfindungsgemäße Gitter-Substrat bietet besondere Vorteile für die Einstellung der anisotropen und/oder örtlich veränderlichen Nachgiebigkeit und die effektive Ausübung der Attraktionskraft der Zellen. Die Nachgiebigkeit des GitterSubstrats kann durch die Verteilung, die Ausrichtung, das Material und/oder die Dimensionen (Durchmesser, Länge) der Gitterelemente eingestellt werden.

Das erfindungsgemäße Substrat mit Deformationsbereichen und/oder inhomogen verteilten Kraftangriffspunkten stellt vorteilhafterweise ein neues Werkzeug der Zellbiologie dar, mit dem Zellen aufgrund endogener Zellkräfte dreidimensionale Zellanordnungen bilden können. Die Form, Struktur und/oder Zusammensetzung der Zellanordnung kann durch das Substrat reproduzierbar vorgegeben werden.

Gemäß der Erfindung ist die mindestens eine Hauptdeformationsrichtung so gewählt, dass das deformierte Substrat einen Hohlraum bildet. Vorteilhafterweise kann damit eine in natürlichem Gewebe oder in Organen gebildete Nische, wie zum Beispiel eine Drüsenstruktur oder ein Gefäß passend nachgebildet werden. So sind gemäß weiteren Alternativen der Erfindung eine Schlauchform oder eine Kugelform vorgesehen, da diese an die Topologie natürlicher Zellstrukturen angepasst sind. Alternativ sind andere Raumformen, wie zum Beispiel Spiralen oder kompakte Geometrien, wie zum Beispiel Kugeln, Ellipsoide oder Toroide formbar.

Gemäß einer weiteren vorteilhaften Ausführungsform der Erfindung kann das Substrat mindestens ein Substratteil aufweisen, dessen Lage und/oder Orientierung relativ zu dem übrigen Substrat, zum Beispiel weiteren Substratteilen oder einem Substratkörper durch die Deformation verändert wird. Besonders bevorzugt umfasst das Substrat mehrere Substratteile, insbesondere Substratlagen (Substratschichten), die durch die Verformung des mindestens einen Substratteils verschieden deformierbar und/oder voneinander trennbar sind. Vorteilhafterweise wird damit die Variabilität bei der Formung der Zellanord nung als gekrümmte Zellschicht oder als dreidimensionaler Zellhaufen weiter vergrößert.

Besonders bevorzugt umfasst das Substrat einen Stapel von Substratlagen. Die Substratlagen, zum Beispiel aus Kunststofffolien, liegen übereinander. Erfindungsgemäß kann die Zellanordnung in einem ersten Schritt auf der obersten Substratlage angeordnet werden, die sich unter der Wirkung der Attraktionskraft der Zellen deformiert. Die deformierte Substratlage legt die Oberfläche der darunter liegenden Substratlage frei, welche nach einer Anordnung von Zellen in einem zweiten Schritt ebenfalls deformiert wird. Die Anordnung der Zellen auf der tiefer liegenden Substratlage kann durch die natürliche Zellwanderung oder durch die Deposition weiterer Zellen erfolgen. Im Ergebnis werden die Substratlagen verschieden verformt, wobei zwischen den Substratlagen Zellen angeordnet sind, die einen dreidimensionalen Zellhaufen bilden. Alternativ können Substratlagen vom übrigen Substrat getrennt und weiteren zellbiologischen Verfahren unterzogen werden.

Vorteilhafterweise kann das erfindungsgemäße Verfahren zur Bildung einer dreidimensionalen Zellanordnung mit weiteren Verfahren der Zellbiologie kombiniert werden. Beispielsweise kann eine Kultivierung der Zellen, die insbesondere ein Wachstum der Zellanordnung und/oder eine Differenzierung der Zellen umfasst, vorgesehen sein. Besonders bevorzugt erfolgt die Kultivierung der Zellen auf dem noch undeformierten Substrat und/oder während der Deformation des Substrats mit der Formung der Zellanordnung. Vorteilhafterweise kann die erfindungsgemäße Bildung einer dreidimensionalen Zellanordnung eine erste Phase einer Kultivierung in einer herkömmlichen Kultivierungseinrichtung, insbesondere auf einem ebenen Substrat, und eine zweite Phase umfassen, in der die gewünschte dreidimensionale Form der Zellanordnung gebildet wird. Vorzugsweise erfolgt die Deformation des Substrats erst nach einer Kultivierung der Zellen auf der Substratoberfläche, besonders bevorzugt nach einem vollständigen Aufwachsen der Zellen auf der Substratoberfläche oder der Bildung einer geschlossenen Zellschicht auf dem Substrat. Somit werden die Zellen zunächst zweidimensional propagiert und anschließend die Zellanordnung in die gewünschte Form gebracht. Dabei wird die Form vorzugsweise durch das Substrat geprägt.

Gemäß einer weiteren Variante der Erfindung kann die Bildung der dreidimensionalen Zellanordnung mit einer Zufuhr von weiteren Zellen zu der Zellanordnung kombiniert werden. Die dreidimensionale Zellanordnung kann als Kultivierungssubstrat für weitere Zellen verwendet werden. Beispielsweise können Stammzellen auf einem dreidimensionalen Gebilde differenzierter Zellen deponiert und einer weiteren Kultivierung, insbesondere Differenzierung unterzogen werden.

Des Weiteren kann nach der Bildung der dreidimensionalen Zellanordnung diese von dem deformierten Substrat getrennt werden. Wenn eine Auflösung des Substrats und/oder eine mechanische Entfernung des Substrats von der Zellanordnung vorgesehen ist, wird vorteilhafterweise eine dreidimensionale Zellanordnung gebildet, die ausschließlich aus biologischen Zellen besteht und frei von nicht-natürlichen Substratmaterialien ist.

Gemäß einer weiteren Ausführungsform der Erfindung kann die Bildung von mehreren dreidimensionalen Zellanordnungen unter Verwendung getrennter Substrate und eine anschließende Kombination der separat hergestellten Zellstrukturen vorgesehen sein. Vorteilhafterweise können damit die Größe und Form der Zellanordnung frei gewählt werden. Dies ist insbesondere für Implantationsanwendungen von Vorteil.

Gemäß einer weiteren Ausführungsform der Erfindung kann die Wirkung der Attraktionskraft der Zellen durch eine zusätzliche Deformationskraft unterstützt werden, die eine äußere mechanische Kraft und/oder eine innere Reaktionskraft im Substratmaterial umfasst. Die äußere mechanische Kraft wird mit einem Deformationswerkzeug ausgeübt, das im Unterschied zu herkömmlichen Techniken keine flächige Berührung des Substrats erfordert. Es ist ausreichend, wenn mit dem Werkzeug an einzelnen Positionen des Substrats die äußere mechanische Kraft ausgeübt wird. Die innere Reaktionskraft im Substratmaterial umfasst zum Beispiel eine innere Spannung, die durch die Struktur des Substratmaterials erzeugt wird. Vorzugsweise ist eine Aktivierung der inneren Reaktionskraft durch eine mechanische, chemische, thermische, elektrische und/oder optische Wirkung vorgesehen.

Die Variabilität der Gestaltung des erfindungsgemäßen Substrats stellt einen weiteren Vorteil der Erfindung dar. Beispielsweise ist das Substrat aus Kunststoff oder Metall hergestellt. Biokompatible und chemisch inerte Materialien werden bevorzugt. Wenn das Substrat aus einem resorbierbaren Material, z. B. Fibrin gebildet ist, kann das Substrat vorteilhafterweise nach der Formung der dreidimensionalen Zellanordnung aufgelöst werden.

Gemäß einer weiteren Ausführungsform der Erfindung kann die Substratoberfläche eine Modifizierungsschicht tragen. Die Modifizierungsschicht ist aus mindestens einer biologisch wirksamen Substanz, wie zum Beispiel einem Differenzierungsfaktor oder einer pharmakologisch wirksamen Substanz oder biologischen Zellen gebildet. Vorteilhafterweise ermöglicht die Modifizierungsschicht eine Beeinflussung der Zellen in der Zellanordnung.

Gemäß einem weiteren Gesichtspunkt wird die o. g. Aufgabe der Erfindung durch eine Zusammensetzung eines biegsamen Substrats mit einer Zellanordnung biologischer Zellen gelöst, wobei das Substrat unter der Wirkung der Attraktionskraft (F_{Z}) der Zellen deformiert ist.

Die Verwendung eines biegsamen Substrats zur Bildung einer dreidimensionalen Zellanordnung stellt einen weiteren unabhängigen Gegenstand der Erfindung dar.

Weitere Einzelheiten und Vorteile der Erfindung werden aus der Beschreibung der beigefügten Zeichnungen ersichtlich. Es zeigen:
- Figuren 1 bis 3:: schematische Illustrationen der erfindungsgemäßen Bildung einer dreidimensionalen Zellanordnung;
- Figuren 4 bis 9:: weitere Ausführungsformen erfindungsgemäßer Substrate;
- Figuren 10 und 11:: weitere Ausführungsformen erfindungsgemäßer Substrate mit mehreren Substratteilen;
- Figur 12:: eine schematische Illustration einer Kultivierungseinrichtung; und
- Figur 13:: eine Illustration eines herkömmlichen Verfahrens zur Bildung einer dreidimensionalen Zellanordnung (Stand der Technik).

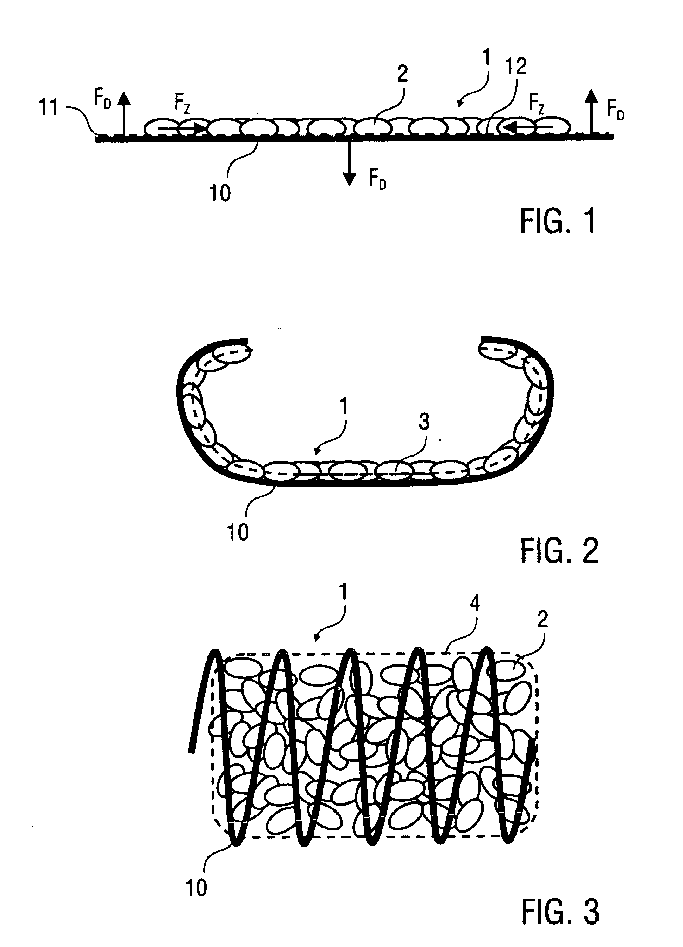

In Figur 1 ist eine erste Ausführungsform eines erfindungsgemäßen Substrats 10 in schematischer Schnittansicht illustriert. Das Substrat 10 besteht aus einem biegeweichen, elastisch oder plastisch deformierbaren Material, wie zum Beispiel Kunststoff, Zellulose, einem Polymer, einem textilen Material, wie z. B. einem Gewebe, einem Gestrick o. dgl., einem Edelmetall oder einem resorbierbaren Material. Das Substrat hat eine zweidimensionale (flächige) Erstreckung, so dass die Form einer Folie (Schicht, Bogen oder Plättchen) oder eines Gitters (Gerüst, Netz) gebildet wird. Das Substrat 10 weist auf einer Oberfläche 11, die zur Aufnahme der Zellanordnung 1 aus biologischen Zellen 2 vorgesehen ist, Kraftangriffspunkte 12 auf. Die Kraftangriffspunkte 12 der Substratoberfläche 11 umfassen zum Beispiel aufgerauhte Bereiche oder chemisch modifizierte Bereiche, die mit biologisch wirksamen Molekülen belegt sind, die mit Zelloberflächenrezeptoren Bindungen bilden. Die Form der Bereiche kann frei gewählt werden. Die Kraftangriffspunkte 12 können z. B. Kreisflächen, Rechtecke oder gerade oder gekrümmte Linien umfassen.

Die Dimensionen des Substrats 10 und die Form und Ausrichtung der Kraftangriffspunkte 12 sind in Abhängigkeit von der gewünschten Deformation des Substrats 10 gewählt. Die Dicke des Substrats ist so gewählt, dass eine genügend geringe Biegesteifigkeit zur Deformation des Substrats 10 unter der Wirkung der Attraktionskraft (F_{Z}) der Zellen erzielt wird. Die Auswahl einer Dicke kann insbesondere durch eine Versuchsreihe mit verschiedenen Schichtdicken unter den konkreten Anwendungsbedingungen realisiert werden. Entlang der Ausdehnung des Substrats 10 kann die Dicke zur Bildung von Deformationsbereichen erhöhter Biegsamkeit (Biegebereich) eine verminderte Dicke aufweisen. Typischerweise ist die Dicke des Substrats 10 für Kunststoff- oder Metall-Substrate im Bereiche unterhalb 10 nm bis 3 mm, insbesondere unterhalb 100 µm gewählt. Die laterale Dimension (Seitenlänge, Umfang) und Form des Substrats 10 wird ebenfalls in Abhängigkeit von der konkreten Anwendung gewählt.

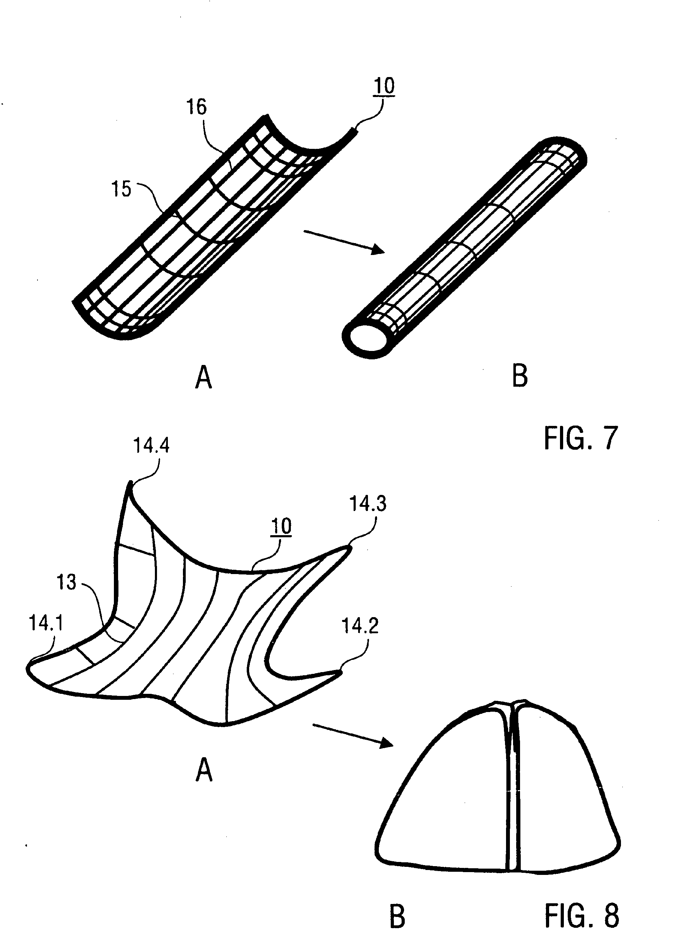

Figur 1 zeigt das Substrat 10 im Zustand vor Beginn der erfindungsgemäßen Deformation. In diesem Zustand hat das Substrat 10 vorzugsweise eine ebene Form, insbesondere mit einer ebenen Substratoberfläche. Abweichend von der in Figur 1 gezeigten Variante kann das Substrat 10 bereits vor der Deformation eine gekrümmte Form aufweisen (siehe z. B. Figur 7).

Die Zellen 2 sind adhärent auf der Substratoberfläche 11 angeordnet. Die Zellen 2 umfassen zum Beispiel tierische Zellen, insbesondere differenzierte Zellen, wie zum Beispiel Fibroblasten, Muskelzellen, Epithelzellen und/oder Endothelzellen, oder Vorläuferzellen, wie zum Beispiel hämatopoetische Precursorzellen, Progenitorzellen und/oder Blastenzellen, oder embryonale oder adulte Stammzellen. Die Zellen 1 bilden die schichtförmige Zellanordnung 2, die eine geschlossene Bedeckung der Substratoberfläche 11 bildet. Die schichtförmige Zellanordnung 2 kann mehrere Zell-Lagen umfassen. Die Dicke der Zellanordnung 2 beträgt zum Beispiel 1 mm. Die adhärenten Zellen 1 bilden zwischenmolekulare Wechselwirkungen zwischen der Zellmembran und der Substratoberfläche 11. Die Zellmembran bildet Adhäsionskontakte mit der Substratoberfläche 11, die wegen der laufenden Umordnung des Zytoskeletts der Zellen einer wiederholten Veränderung unterliegen. Gleichzeitig erfolgen zwischenmolekulare Wechselwirkungen zwischen den Zellen 2, die zu Kohäsionsbindungen zwischen den Zellen führen. Die Erfinder haben festgestellt, dass die Kohäsionsbindungen ausreichend stark sein können, um die Attraktionskraft zu erzeugen, unter deren Wirkung das Substrat 10 deformiert wird. Die tangential zur Substratoberfläche ausgeübte Attraktionskraft ist in Figur 1 schematisch mit F_{Z} gezeigt. Zusätzlich zur Attraktionskraft F_{Z} kann auf das Substrat 10 an mindestens einer Position eine lokal wirkende Deformationskraft F_{D} mit einem äußeren Werkzeug (nicht dargestellt) ausgeübt werden, um die Wirkung der Attraktionskraft zu unterstützen.

Zur Durchführung des erfindungsgemäßen Verfahrens wird das Substrat 10 in einer Kultivierungseinrichtung in einer Kultivierungsflüssigkeit angeordnet. Einzelheiten der Kultivierungseinrichtung werden unten unter Bezug auf Figur 12 beschrieben. Auf das Substrat 10 werden Zellen 2 aufgebracht und einer Kultivierung unterzogen, bis die Zellanordnung 1 gebildet ist. Die Kultivierung erfolgt unter üblichen Kultivierungsbedingungen, wie sie aus den Techniken der Zellbiologie bekannt sind. Sobald die Attraktionskraft der Zellen 2 ausreichend groß ist, beginnt die Deformation des Substrats 10. In Abhängigkeit von der lokalen Verteilung der Nachgiebigkeit des Substrats 10 wird dieses einer Krümmung oder einer Faltung unterzogen, wie beispielhaft in den Figuren 2 und 3 gezeigt ist. Die Geschwindigkeit der Deformation wird durch die Geschwindigkeit der Umordnung molekularer Zellkontakte in der Zellanordnung und/oder die Kultivierungsgeschwindigkeit der Zellanordnung bestimmt. Die komplette Deformation kann beispielsweise in einem Zeitbereich von Tagen bis Wochen erfolgen. Die Deformation endet insbesondere, wenn eine vom Substrat gebildete Gegenkraft ein Gleichgewicht mit der von den Zellkräften ausgeübten Attraktionskraft bildet. Um ein gewünschtes Zieles zu erreichen (z. B. eine Krümmung, eine Dicke der Zellanordnung, eine Röhrenform, ein Hohlraum, o. dgl.), wird das Material und die Geometrie des Substrates und insbesondere der Kraftangriffspunkte geeignet gewählt. Alternativ oder zusätzlich kann die Deformation durch eine Auflösung des Substrates oder eine Entnahme des Substrates mit den Zellen aus einem Kultivierungsmedium (Zellproliferationsmedium) beendet werden.

Mit einem Substrat 10, das einen Gradienten einer zu den Rändern hin sich vermindernden Nachgiebigkeit aufweist, ergibt die Attraktionskraft der Zellen die schematisch in Figur 2 illustrierte Substratkrümmung. Die Zellanordnung 1 bildet eine gekrümmte Zellschicht 3, die sich entlang einer im Raum gekrümmten Fläche (gestrichelte Linie) erstreckt. Mit einem Substrat 10, das auf beiden Oberflächen Kraftangriffspunkte und eine Anordnung paralleler, linienförmiger Biegebereiche aufweist, ergibt sich die in Figur 3 gezeigte Substratfaltung. Die Zellen 2 der Zellanordnung 1 bilden einen dreidimensionalen Zellhaufen 4. Im Zellhaufen 4 ist das gefaltete Substrat 10 enthalten. Bei Verwendung eines resorbierbaren Materials kann das gefaltete Substrat 10 während der weiteren Kultivierung der Zellen 2 im Zellhaufen 4 aufgelöst werden.

Die gerichtete Deformation des Substrats ist auch möglich, wenn die Zellen auf beiden Oberflächen des Substrats (z. B. eines Gittersubstrats) gewachsen sind. Die Krümmungsrichtung kann sich aus Unterschieden im Bewuchs beider Oberflächen ergeben. Diese Unterschiede können zufällig sein oder gezielt durch die Bereitstellung verschiedener Mengen der Zellen auf den Oberflächen eingestellt werden. Des Weiteren kann die Attraktionskraft der Zellen auf einer der Oberflächen durch eine Zufuhr von Partikeln, welche die Zellkräfte beeinflussen, insbesondere von Nano- oder Mikrobeads, auf einer der Oberflächen verändert werden, um die Krümmungsrichtung zu beeinflussen. Schließlich kann die Krümmungsrichtung durch Materialeigenschaften des Substrats (insbesondere Einstellung der Hauptdeformationsrichtung) eingestellt werden.

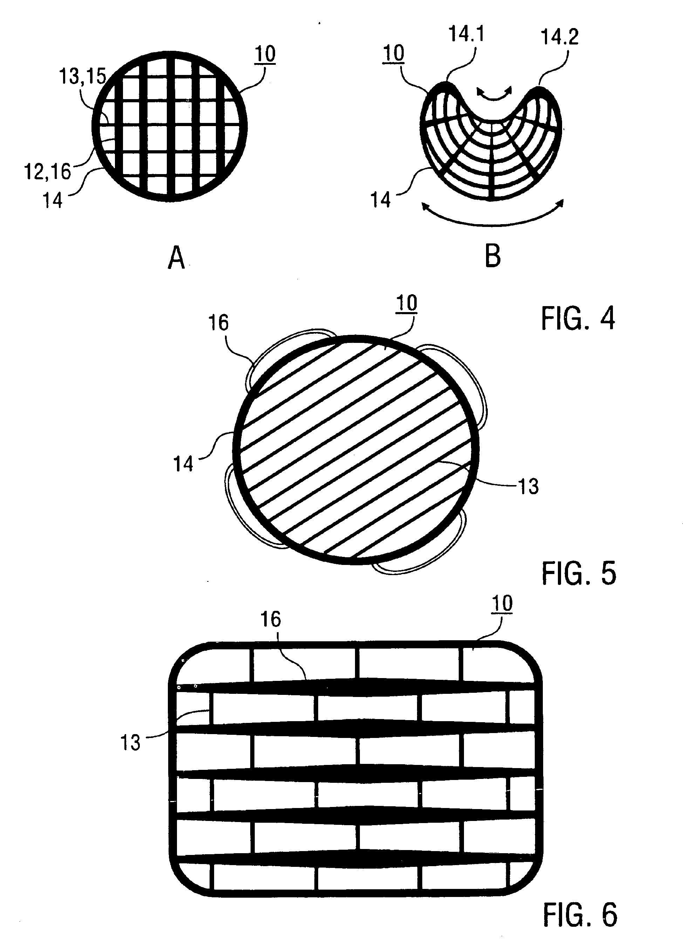

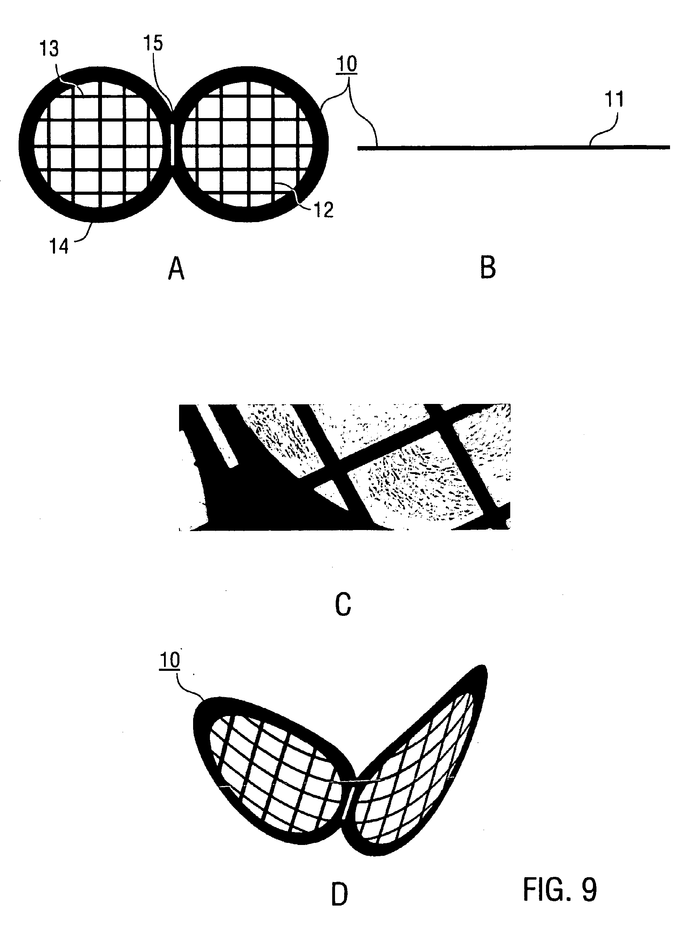

Weitere Beispiele erfindungsgemäßer Substrate 10 mit einer Gitterstruktur (Gitter-Substrate) sind in den Figuren 4 bis 9 illustriert. Allgemein umfasst ein Substrat 10 mit einer Gitterstruktur eine Vielzahl von Gitterelementen 13, die von einem Gitterrahmen 14 umgeben sind (siehe zum Beispiel Figur 4A). Die Form des Gitterrahmens 14 kann eine einfache geometrische Form (zum Beispiel Kreis oder Rechteck, siehe Figuren 4 bis 7) oder eine komplexere Form (siehe Figuren 8, 9) haben. Aus Klarheitsgründen sind die Gitter-Substrate außer in Figur 9C jeweils ohne die Zellanordnung gezeigt.

Die Gitterelemente 13 und der Gitterrahmen 14 sind so gebildet, dass das Substrat 10 eine vorbestimmte Hauptdeformationsrichtung erhält. Gemäß Figur 4A sind beispielsweise steifere (dickere) Gitterelemente in einer ersten Richtung (Längsrichtung) vorgesehen, die zugleich die Kraftangriffspunkte 12 und die Versteifungsbereiche 16 bilden. Des Weiteren sind Gitterelemente 13 (Biegebereiche 15) mit geringerer Biegesteifigkeit in einer zweiten Richtung (Querrichtung) vorgesehen. Somit weist das Gitter-Substrat 10 gemäß Figur 4A in Querrichtung eine geringere Biegesteifigkeit auf als in Längsrichtung.

Mit der Bildung einer geschlossenen Zellanordnung auf dem Gitter-Substrat 10 wird dieses deformiert. Entsprechend der Richtung der verminderten Biegesteifigkeit ergibt sich eine Krümmung. Die Krümmung kann derart erfolgen, dass sich gegenüberliegende Ränder 14.1, 14.2 des Gitterrahmens 14 berühren und eine geschlossene, dreidimensionale Form, z. B. eine Tasche oder Nische gebildet wird (Figur 4B).

Figur 5 zeigt ein weiteres Beispiel eines Gitter-Substrats 10 mit einer Vielzahl paralleler Gitterelemente 13 und einem Gitterrahmen 14, an dessen äußerem Rand Versteifungsbereiche 16 vorgesehen sind. Die Gitterelemente 13 bilden zugleich die Kraftangriffspunkte des Gitter-Substrats 10. Die Versteifungsbereiche 16 dienen der Formbeeinflussung bei der Deformation des Substrats 10. Bei dem Gitter-Substrat 10 gemäß Figur 5 sind durch die komplexe Überlagerung der Wirkung der Gitterelemente 13 und der Versteifungsbereiche 16 mehrere Hauptdeformationsrichtungen gegeben.

Die Versteifungsbereiche 16 können alternativ oder zusätzlich an den Gitterelementen 13 vorgesehen sein, wie beispielhaft in Figur 6 gezeigt ist. Die Gitterelemente 13 haben in einer ersten Richtung (Längsrichtung) eine geringe Dicke und entsprechend eine geringe Biegesteifigkeit. In einer zweiten Richtung (Querrichtung) weisen die Gitterelemente eine Verdickung zur Bildung der Versteifungsbereiche 16 auf. Es ergibt sich eine erhöhte Biegesteifigkeit. Die Hauptdeformationsrichtung des Substrats 10 gemäß Figur 6 ist somit durch die geringe Biegesteifigkeit in Längsrichtung vorgegeben.

In Figur 7A ist beispielhaft eine Ausführungsform eines Gitter-Substrats 10 gezeigt, das bereits vor der Ausübung der Attraktionskraft der Zellen eine gekrümmte Vorform aufweist. Das Gitter-Substrat 10 bildet den Teil eines Mantels eines geraden Kreiszylinders. Die Gitterelemente in axialer Richtung besitzen eine größere Dicke (Versteifungsbereiche 16) als die Gitterelemente in azimutaler Richtung (Biegebereiche 15). Nach Bewuchs mit einer Zellanordnung wird das Gitter-Substrat 10 durch die Attraktionskraft weiter gekrümmt, bis sich die Schlauch- oder Rohrform gemäß Figur 7B ergibt. Die in Figur 7 gezeigte Ausführungsform der Erfindung hat besondere Vorteile für die Nachbildung von biologischen Organen oder Organbestandteilen, die zur Flüssigkeitsleitung vorgesehen sind, wie zum Beispiel von Röhren oder Gefäßen, insbesondere Blutgefäßen.

Die in Figur 8 gezeigte Ausführungsform des Gitter-Substrats 10 besitzt eine komplexere Form mit mehreren Hauptdeformationsrichtungen. Unter der Wirkung der Attraktionskraft der Zellen wird der Gitterrahmen 14 des Gitter-Substrats 10 so gekrümmt, dass einander gegenüberliegende Randbereiche, insbesondere die Eckbereiche 14.1 bis 14.4 sich gegenseitig berühren (Figur 8B). Vorteilhafterweise wird damit ein Hohlraum geschaffen, der als Substratnische für die Kultivierung von Zellen der auf dem Substrat angeordneten Zellanordnung oder von zusätzlich zugeführten Zellen, zum Beispiel einer Stammzelle, verwendbar ist.

Figur 9 zeigt ein weiteres Gitter-Substrat 10 mit einer komplexen äußeren Form in verschiedenen Phasen der erfindungsgemäßen Substratdeformation (A: Draufsicht, B, D: Schnittansicht, C: photographische Teilansicht, E: Perspektivansicht). In einer ersten Phase vor Beginn der Deformation hat das Gitter-Substrat 10 eine ebene Form (Figuren 9A, B). Der Gitterrahmen 14 ist aus zwei Kreisen zusammengesetzt, zwischen denen ein Biegebereich 15 mit verminderter Biegesteifigkeit gebildet ist. Die Gitterelemente 13 sind als gleichförmiges Rechteckgitter mit gleichen Dicken angeordnet. Nach Bewuchs mit einer Zellanordnung mit Stammzellen einer Ratte wurde eine Krümmung des Gitter-Substrats 10 um den Biegebereich 15 erhalten (Figuren 9C, 9D).

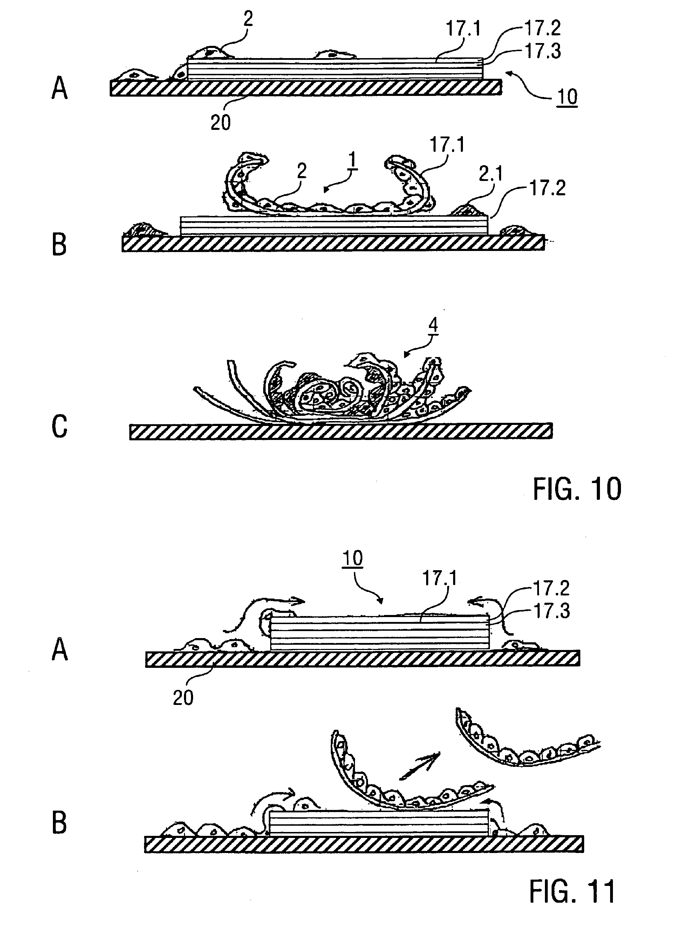

Die Figuren 10 und 11 zeigen zwei Ausführungsformen der Erfindung, bei denen das Substrat 10 einen Stapel mit einer Vielzahl von Substratlagen 17.1, 17.2, 17.3, ... umfasst. Die Substratlagen 17.1, 17.2, 17.3, ... umfassen zum Beispiel Kunststofffolien, die lediglich durch Adhäsionskräfte zusammengehalten werden.

Die in Figur 10 gezeigte Ausführungsform der erfindungsgemäßen Bildung einer dreidimensionalen Zellanordnung 1 umfasst die folgenden Schritte. Zunächst erfolgt die Deposition von Zellen 2 auf der obersten Substratlage 17.1. Hierbei werden an sich bekannte Depositionstechniken, wie zum Beispiel ein Auftropfen mit einer Pipette oder vorzugsweise wie dargestellt die natürliche Wanderungsbewegung adhärenter Zellen ausgenutzt. Die adhärenten Zellen wandern gemäß Figur 10A von einem Träger 20 auf die oberste Substratlage 17.1.

Sobald die Zahl der Zellen 2 in der Zellanordnung 1 auf der obersten Substratlage 17.1 so groß ist, dass eine ausreichend große Attraktionskraft ausgeübt wird, krümmt sich die oberste Substratlage 17.1. In dieser zweiten Phase (Figur 10B) wandern weitere Zellen des gleichen oder eines anderen Typs auf die zweite Substratlage 17.2, an der wiederum eine Deformation erfolgt. Im weiteren Verfahrensverlauf erhält das schichtförmige Substrat 10 durch den Bewuchs der Zellen und die von den Zellen ausgeübte Attraktionskraft eine vorbestimmte Form. Die Form wird durch die Verteilung der Biegesteifigkeit in den einzelnen Substratlagen definiert. Im Ergebnis wird die in Figur 10 beispielhaft gezeigte Zellanordnung 1 in Form eines komplexen dreidimensionalen Zellhaufens 4 (Zellaggregat) bestehend aus Schichten eines einzigen Zelltyps oder unterschiedlicher Zelltypen gebildet. Vorteilhafterweise können derartige Zellanordnungen insbesondere als Kokultursysteme oder als Gewebemodell beim Tissue Engineering verwendet werden.

Figur 11 zeigt eine alternative Variante der Verwendung eines aus mehreren Substratlagen 17.1, 17.2, 17.3, ... bestehenden Substrats 10. Die Zellen 2 wandern zunächst auf die oberste Substratlage 17.1 (Figur 11A), die sich nach ausreichendem Bewuchs deformiert und von der darunter liegenden Substratlage 17.2 trennen lässt (Figur 11B).

Mit dem in Figur 11 illustrierten Verfahren können geschichtete Substrate 10 mit mehreren Substratlagen zur Herstellung von mit Zellen besiedelten Keimen für eine trypsinfreie Propagation verwendet werden. Die besiedelten Substratlagen können als Keime zur weiteren Vermehrung der Zellen ohne die Verwendung von Trypsin vom übrigen Substrat 10 getrennt und in neue Kultivierungsgefäße überführt werden.

Figur 12 zeigt beispielhaft eine Kultivierungseinrichtung 100, mit der das erfindungsgemäße Verfahren vorzugsweise realisiert wird. Das erfindungsgemäße Substrat 10 ist auf einem Träger 20 in einem Kultivierungsgefäß 40 angeordnet, das ein flüssiges Kultivierungsmedium 41 enthält. Unter der Wirkung der Attraktionskraft in der Zellanordnung 1 wird das Substrat 10 deformiert, wie im rechten Teilbild von Figur 12 gezeigt ist. Des Weiteren zeigt Figur 12 ein Werkzeug 30 zur Ausübung einer äußeren Deformationskraft auf das Substrat 10. Das Werkzeug 30 umfasst zum Beispiel zwei spitzenförmige Werkzeugelemente 31, mit denen an Rändern des Substrats 10 eine Kraft ausgeübt wird, mit der die Attraktionskraft der Zellen unterstützt wird. Das Werkzeug 30 ist mit einem Positioniersystem 32 und einer Steuereinrichtung 33 verbunden.

Mit den oben beschriebenen Verformungen erfindungsgemäßer Substrate und/oder die Kombination von mehreren deformierten Substraten können erfindungsgemäß vielschichtige Zellsysteme gebildet werden, die vorteilhafterweise zahlreiche Anwendungen in der Zellbiologie, der medizinischen Therapie oder dem Tissue Engineering haben. Beispielsweise können multizelluläre Kugeln (Figur 4) als Gewebemodelle für so genannte organoide Körper verwendet werden. Hohlformen können biologische Wirkstoffe, biologische Materialien, wie zum Beispiel Differenzierungsfaktoren oder Zellbestandteile einschließen. Für Tissue Engineering-Aufgaben können organoide Strukturen nachgebildet werden.

Die Verwendung langsam resorbierbarer Materialien insbesondere als Gitter-Substrat ermöglicht die Herstellung dreidimensionaler Zellsysteme mit einer definierten Form, die nach der Resorption des Materials ihre Form beibehalten. Derartige dreidimensionale Zellanordnungen eignen sich insbesondere zur Implantation in einen Organismus.

Des Weiteren lassen sich durch die Kombination von separat hergestellten Zellstrukturen dreidimensionale Zellanordnungen mit aus verschiedenen Zelltypen bestehenden Schichten erzeugen, die insbesondere als Kokultursystem oder Gewebemodell dienen können. Derartige Zellanordnungen können manuell kombinier oder durch Faltung geschichteter Substrate erzeugt werden (Figur 10). Ferner kann durch die Beschichtung des erfindungsgemäßen Substrats mit einer Modifizierungsschicht, insbesondere mit bioaktiven Molekülen eine Differenzierung von Zellen induziert, eine beschichtungsspezifische Besiedlung mit verschiedenen Zelltypen erzeugt oder eine künstliche Stammzellnische geschaffen werden.

Die in der vorstehenden Beschreibung, den Zeichnungen und den Ansprüchen offenbarten Merkmale der Erfindung können einzeln oder in Kombination für die Verwirklichung der Erfindung in ihren verschiedenen Ausgestaltungen von Bedeutung sein.

## Patentansprüche

1. Verfahren zur Bildung einer dreidimensionalen Zellanordnung (1) biologischer Zellen (2), mit den Schritten:
- Bereitstellung der Zellen (2) auf einem biegsamen Substrat (10), und
- Deformation des Substrats (10), die durch eine von den Zellen (2) auf das Substrat (10) ausgeübte Attraktionskraft (F_{Z}) bewirkt wird, wobei das Substrat (10) eine vorbestimmte geometrische Anordnung von Kraftangriffspunkten (12) aufweist,
die zur Ausübung der Attraktionskraft (Fz) von den Zellen (2) auf das Substrat (10) eingerichtet sind,
**dadurch gekennzeichnet, dass**
- das Substrat (10) eine vorbestimmte geometrische Anordnung von Deformationsbereichen, die jeweils mindestens einen Biegebereich (15) und/oder mindestens einen Versteifungsbereich (16) umfassen, und eine Nachgiebigkeit aufweist, die anisotrop und örtlich veränderlich ist, und
- die Deformation eine gerichtete Deformation des Substrats (10) mit mindestens einer vorbestimmten Hauptdeformationsrichtung umfasst, die durch die Anordnung der Deformationsbereiche und der Kraftangriffspunkte (12) des Substrats (10) bestimmt ist, wobei
- die Deformation eine Faltung des Substrats (10) derart umfasst, dass die Zellanordnung einen dreidimensionalen Zellhaufen (4) bildet, und
- die Deformationsbereiche so angeordnet sind, dass das unter der Wirkung der Attraktionskraft (F_{Z}) deformierte Substrat (10) einen Hohlraum, eine Schlauchform und/oder eine Kugelform bildet.

2. Verfahren nach Anspruch 1, bei dem die Zellen (2) auf einer Substratoberfläche (11) des Substrats (10) adhärent angeordnet sind und eine geschlossene Zellschicht bilden.

3. Verfahren nach mindestens einem der vorhergehenden Ansprüche, bei dem die Deformation des Substrats (10) eine Verformung von mindestens einem Substratteil, dessen Lage und/oder Ausrichtung relativ zu dem übrigen Substrat (10) durch die Deformation verändert wird, und/oder eine Verformung von mindestens einer flächigen Substratlage (17.1, 17.2, 17.3, ...) umfasst, wobei die mindestens eine Substratlage (17.1, 17.2, 17.3, ...) verformt und/oder von dem übrigen Substrat (10) getrennt wird.

4. Verfahren nach mindestens einem der vorhergehenden Ansprüche, mit mindestens einem der Schritte:
- Kultivierung der Zellen (2) in der Zellanordnung (1),
- Zufuhr von weiteren Zellen (2.1) zu der Zellanordnung (1),
- Auflösung und/oder Entfernung des Substrats (10), und
- Verbindung von mehreren deformierten Substraten (10).

5. Verfahren nach Anspruch 4, bei dem die Deformation des Substrats (10) und die Formung der Zellanordnung (1) während oder nach der Kultivierung der Zellen erfolgt.

6. Verfahren nach mindestens einem der vorhergehenden Ansprüche, bei dem auf das Substrat (10) mindestens eine zusätzliche Deformationskraft wirkt, die eine äußere mechanische Kraft und/oder eine innere Reaktionskraft im Material des Substrats umfasst.

7. Verfahren nach mindestens einem der vorhergehenden Ansprüche, bei dem das Substrat (10)
- aus Kunststoff, Metall und/oder einem resorbierbarem Material gebildet ist,
- eine geschlossene Substratoberfläche (11) aufweist,
- eine durch Löcher oder Vertiefungen unterbrochene Substratoberfläche (11) aufweist,
- eine Gitterstruktur aufweist, und/oder
- eine Modifizierungsschicht trägt.

8. Substrat (10), das aus einem biegsamen Material gebildet ist und eine Substratoberfläche (11) zur Anhaftung einer Zellanordnung (1) biologischer Zellen (2) aufweist, wobei
- die Substratoberfläche (11) eine Vielzahl von Kraftangriffspunkten (12) aufweist, die zur Ausübung einer Attraktionskraft (Fz) eingerichtet sind, die von den Zellen (2) auf das Substrat (10) übertragbar ist, und
- das Substrat (10) eine Nachgiebigkeit derart aufweist, dass das Substrat (10) unter der Wirkung der Attraktionskraft (Fz) deformierbar ist
**dadurch gekennzeichnet, dass**
- die Nachgiebigkeit anisotrop und örtlich veränderlich ist,
- das Substrat (10) eine vorbestimmte geometrische Anordnung von Deformationsbereichen aufweist, die jeweils mindestens einen Biegebereich (15) und/oder mindestens einen Versteifungsbereich (16) derart umfassen, dass das deformierte Substrat (10) einen Hohlraum, eine Schlauchform und/oder eine Kugelform bildet, und
- das Substrat (10) mindestens eine vorbestimmte Hauptdeformationsrichtung aufweist, die durch die geometrische Anordnung des mindestens einen Biegebereiches (15), des mindestens einen Versteifungsbereiches (16) und der Kraftangriffspunkte (12) bestimmt ist.

9. Substrat nach Anspruch 8, das
- zumindest in einem Teilbereich eine Gitterstruktur mit einer Vielzahl von Gitterelementen (13) aufweist, welche die Kraftangriffspunkte (12) bilden, und/oder
- zumindest in einem Teilbereich eine geschlossene Substratoberfläche (11) aufweist, auf der die Kraftangriffspunkte (12) gebildet sind.

10. Substrat nach mindestens einem der Ansprüche 8 bis 9,
- das mindestens ein deformierbares Substratteil aufweist, dessen Lage und/oder Ausrichtung relativ zu dem übrigen Substrat (10) veränderlich ist,
- aus Kunststoff, Metall und/oder einem resorbierbarem Material gebildet ist, und/oder
- eine Modifizierungsschicht trägt.

11. Substrat nach Anspruch 10, bei dem
- das mindestens eine deformierbare Substratteil mindestens eine flächige Substratlage (17.1, 17.2, 17.3, ...) umfasst.

12. Substrat nach mindestens einem der Ansprüche 8 bis 11,
- das zur Deformation durch mindestens eine zusätzliche Deformationskraft (F_{D}) eingerichtet ist, die eine äußere mechanische Kraft und/oder eine innere Reaktionskraft im Material des Substrats umfasst.

13. Substrat nach mindestens einem der Ansprüche 8 bis 12, wobei auf der Substratoberfläche (11) die Zellanordnung (1) angeordnet ist und das Substrat (10) unter der Wirkung der Attraktionskraft (F_{Z}) der Zellen (2) deformiert ist.

## Claims

1. Method for forming a three-dimensional cell arrangement (1) of biological cells (2), comprising the steps:
- provision of the cells (2) on a flexible substrate (10), and
- deformation of the substrate (10), the deformation being produced by an attractive force (Fz) exerted by the cells (2) on the substrate (10), wherein the substrate (10) has a predetermined geometric arrangement of force action points (12), which are adapted for exerting the attractive force (F_{Z}) by the cells (2) on the substrate (10),
**characterized in that**
- the substrate (10) has a predetermined geometric arrangement of deformation regions, each of which comprising at least one bending region (15) and/or at least one stiffening region (16), and a flexibility, which is anisotropic and locally variable, and
- the deformation comprises a directional deformation of the substrate (10) with at least one predetermined main deformation direction, which is determined by the arrangement of the deformation regions and the force action points (12) of the substrate (10), wherein
- the deformation comprises a folding of the substrate (10) such that the cell arrangement forms a three-dimensional pile of cells (4), and
- the deformation regions are arranged such that the substrate (10) deformed by the action of the attractive force (Fz) forms a cavity, a tubular shape and/or a spherical shape.

2. Method according to claim 1, wherein the cells (2) are adherently arranged on a substrate surface (11) of the substrate (10) and form a continuous layer of cells.

3. Method according to at least one of the preceding claims, wherein the deformation of the substrate (10) comprises a shape change of at least one substrate part, the position and/or alignment of which relative to the remaining substrate (10) being varied by the deformation, and/or a shape change of at least one planar substrate layer (17.1, 17.2, 17.3, ...), with the at least one substrate layer (17.1, 17.2, 17.3, ...) changing shape and/or being separated from the remaining substrate (10).

4. Method according to at least one of the preceding claims, having at least one of the steps:
- cultivating of the cells (2) in the cell arrangement (1),
- supply of further cells (2.1) to the cell arrangement (1),
- dissolution and/or removal of the substrate (10), and
- joining of a plurality of deformed substrates (10).

5. Method according to claim 4, wherein the deformation of the substrate (10) and the formation of the cell arrangement (1) take place while or after the cells are cultivated.

6. Method according to at least one of the preceding claims, wherein there acts upon the substrate (10) at least one additional deformation force which comprises an external mechanical force and/or an internal reaction force in the material of the substrate.

7. Method according to at least one of the preceding claims, wherein the substrate (10)
- is made from plastic, metal and/or a resorbable material,
- has a continuous substrate surface (11),
- has a substrate surface (11) broken by holes or depressions,
- has a lattice structure, and/or
- carries a modification layer.

8. Substrate (10), which is made from a flexible material and has a substrate surface (11) for the adhesion of a cell arrangement (1) of biological cells (2), wherein
- the substrate surface (11) has a plurality of force action points (12) which are adapted for exerting an attractive force (F_{Z}) that is transmissible from the cells (2) to the substrate (10), and
- the substrate (10) has a flexibility such that the substrate (10) is deformable under the action of the attractive force (F_{z}),
**characterized in that**
- the flexibility is anisotropic and locally variable,
- the substrate (10) has a predetermined geometric arrangement of deformation regions, each of which comprising at least one bending region (15) and/or at least one stiffening region (16) such that the deformed substrate (10) forms a cavity, a tubular shape and/or a spherical shape, and
- the substrate (10) has at least one predetermined main deformation direction that is determined by the geometric arrangement of the at least one bending region (15), the at least one stiffening region (16) and the force action points (12).

9. Substrate according to claim 8, which has
- at least in one section, a lattice structure with a plurality of lattice elements (13) that form the force action points (12), and/or
- at least in one section, a continuous substrate surface (11) on which the force action points (12) are formed.

10. Substrate according to at least one of claims 8 to 9, which
- has at least one deformable substrate part, the position and/or alignment of which relative to the remaining substrate (10) is variable,
- is made from plastic, metal and/or a resorbable material, and/or
- has a modification layer.

11. Substrate according to claim 10, wherein
- the at least one deformable substrate part comprises at least one planar substrate layer (17.1, 17.2, 17.3, ...).

12. The substrate according to at least one of claims 8 to 11,
- which is adapted for deformation by at least one additional deformation force (F_{D}) which comprises an external mechanical force and/or an internal reaction force in the material of the substrate.

13. Substrate according to at least one of claims 8 to 12, wherein the cell arrangement (1) being arranged on the substrate surface (11) and the substrate (10) being deformed under the action of the attractive force (F_{Z}) of the cells (2).

## Revendications

1. Procédé de formation d'un ensemble cellulaire (1) tridimensionnel de cellules biologiques (2), comportant les étapes de :
- mise à disposition des cellules (2) sur un substrat (10) flexible, et
- déformation du substrat (10) qui est entraînée par une force d'attraction (F_{Z}) exercée par les cellules (2) sur le substrat (10), dans lequel le substrat (10) présente une disposition géométrique prédéterminée de points d'application des forces (12), qui sont conçus pour exercer la force d'attraction (Fᵣ) des cellules (2) sur le substrat (10),
**caractérisé en ce que**
- le substrat (10) présente une disposition géométrique prédéterminée de zones de déformation, qui comprennent respectivement au moins une zone flexible (15) et/ou au moins une zone rigide (16), et une souplesse qui peut être modifiée sur le plan anisotrope et local, et
- la déformation comprend une déformation ciblée du substrat (10) avec au moins une direction principale de déformation prédéterminée qui est déterminée par l'ensemble des zones de déformation et des points d'application de forces (12) du substrat (10), dans lequel
- la déformation comprend un pliage du substrat (10) de telle sorte que l'ensemble cellulaire forme un amas cellulaire tridimensionnel (4), et
- les zones de déformation sont conçues de telle sorte que le substrat (10) déformé sous l'effet de la force d'attraction (F_{Z}) forme une cavité, une forme tubulaire et/ou une forme sphérique.

2. Procédé selon la revendication 1, dans le cadre duquel les cellules (2) sont disposées de façon adhérente sur une surface de substrat (11) du substrat (10) et forment une couche cellulaire fermée.

3. Procédé selon au moins l'une des revendications précédentes, dans le cadre duquel la déformation du substrat (10) comprend une déformation d'au moins une partie de substrat, dont la position et/ou l'orientation est modifiée par rapport au substrat (10) restant par la déformation, et/ou une déformation d'au moins une couche de substrat (17.1, 17.2, 17.3,...) plane, dans lequel l'au moins une couche de substrat (17.-1, 17.2, 17.3...) est déformée et/ou séparée du substrat restant (10).

4. Procédé selon au moins l'une des revendications précédentes, comportant au moins l'une des étapes de :
- culture des cellules (2) dans l'ensemble cellulaire (1),
- apport d'autres cellules (2.1) à l'ensemble cellulaire (1),
- détachement et/ou retrait du substrat (10), et
- liaison de plusieurs substrats déformés (10).

5. Procédé selon la revendication 4, dans le cadre duquel la déformation du substrat (10) et la formation de l'ensemble cellulaire (1) s'effectuent pendant ou après la culture des cellules.

6. Procédé selon au moins l'une des revendications précédentes, dans le cadre duquel, sur le substrat (10), agit au moins une force de déformation supplémentaire qui comprend une force mécanique externe et/ou une force réactionnelle interne dans le matériau du substrat.

7. Procédé selon au moins l'une des revendications précédentes, dans le cadre duquel le substrat (10)
- est formé de matière plastique, de métal et/ou d'un matériau résorbable,
- présente une surface de substrat fermée (11),
- présente une surface de substrat (11) interrompue par des trous ou des creux,
- présente une structure grillagée, et/ou
- porte une couche de modification.

8. Substrat (10), qui est formé d'un matériau flexible et présente une surface de substrat (11) pour l'adhérence d'un ensemble cellulaire (1) de cellules biologiques (2), dans lequel
- la surface de substrat (11) présente une multiplicité de points d'application de forces (12), qui sont conçus pour exercer une force d'attraction (F_{z}), qui peut être transmise des cellules (2) au substrat (10), et
- le substrat (10) présente une souplesse telle que le substrat (10) est déformable sous l'effet de la force d'attraction (F_{z}),
**caractérisé en ce que**
- la souplesse est modifiable sur le plan anisotrope et local,
- le substrat (10) présente une disposition géométrique prédéterminée de zones de déformation, qui comprennent respectivement au moins une zone flexible (15) et/ou au moins une zone rigide (16), de telle sorte que le substrat (10) déformé forme une cavité, une forme tubulaire et/ou une forme sphérique, et
- le substrat (10) présente au moins une direction principale de déformation prédéterminée, qui est déterminée par la disposition géométrique de l'au moins une zone flexible (15), de l'au moins une zone rigide (16) et des points d'application de force (12).

9. Substrat selon la revendication 8, qui
- présente au moins dans une zone partielle, une structure grillagée comportant une multiplicité d'éléments de grille (13), qui forment les points d'application de forces (12), et/ou
- présente au moins, dans une zone partielle, une surface de substrat fermée (11), sur laquelle les points d'application de force (12) sont formés.

10. Substrat selon au moins l'une des revendications 8 à 9,
- qui présente au moins une partie de substrat déformable, dont la position et/ou l'orientation par rapport au substrat (10) restant est modifiable,
- qui est formé de matière plastique, de métal et/ou d'un matériau résorbable, et/ou
- qui porte une couche de modification.

11. Substrat selon la revendication 10, dans le cadre duquel
- l'au moins une partie de substrat déformable comprend au moins une couche de substrat (17.1, 17.2, 17.3,...) plane.

12. Substrat selon au moins l'une des revendications 8 à 11,
- qui est conçu pour la déformation par au moins une force de déformation supplémentaire (F_{D}), qui comprend une force mécanique externe et/ou une force réactionnelle interne dans le matériau du substrat.

13. Substrat selon au moins l'une des revendications 8 à 12, dans lequel l'ensemble cellulaire (1) est disposé sur la surface de substrat (11) et le substrat (10) est déformé sous l'effet de la force d'attraction (F_{Z}) des cellules (2).
